(19) Europäisches Patentamt
European Patent Office
Office européen des brevets

(11) **EP 3 677 283 A1**

(12) **EUROPEAN PATENT APPLICATION**
published in accordance with Art. 153(4) EPC

(43) Date of publication:
**08.07.2020 Bulletin 2020/28**

(51) Int Cl.:
*A61K 47/26* (2006.01)    *A61K 9/08* (2006.01)
*A61K 31/282* (2006.01)    *A61K 31/505* (2006.01)
*A61K 31/519* (2006.01)    *A61K 31/704* (2006.01)
*A61K 31/7088* (2006.01)    *A61K 33/24* (2019.01)
*A61K 35/12* (2015.01)    *A61K 45/00* (2006.01)
*A61P 35/00* (2006.01)    *A61P 35/04* (2006.01)

(21) Application number: **18851860.9**

(22) Date of filing: **30.08.2018**

(86) International application number:
**PCT/JP2018/032220**

(87) International publication number:
**WO 2019/045005 (07.03.2019 Gazette 2019/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2017   JP 2017167951**

(71) Applicant: **Tohoku University**
**Sendai-shi, Miyagi 980-8577 (JP)**

(72) Inventors:
• **KODAMA, Tetsuya**
  **Sendai-shi**
  **Miyagi 980-8577 (JP)**
• **MORI, Shiro**
  **Sendai-shi**
  **Miyagi 980-8577 (JP)**

(74) Representative: **Blodig, Wolfgang**
  **Wächtershäuser & Hartz**
  **Patentanwaltspartnerschaft mbB**
  **Weinstrasse 8**
  **80333 München (DE)**

(54) **APPROPRIATE OSMOTIC PRESSURE RANGE OF SOLUTION CONTAINING DRUG EFFECTIVE IN LYMPHATIC DRUG DELIVERY SYSTEM**

(57)    Provided is an optimal intralymphatic preparation containing agents such as a drug, a cell, or a nucleic acid for use in a lymphatic drug delivery system.
    A drug-containing liquid preparation for delivering a chemical to a target lymph node by a lymphatic drug delivery system, wherein the liquid has an osmotic pressure of 700 to 2,700 kPa.

Figure 1

**Description**

TECHNICAL FIELD

The present invention relates to the osmotic pressure of a solution containing a drug that would be useful for lymphatic drug delivery.

BACKGROUND ART

Cancer affects 50% of Japanese people, and 90% of patients with cancer die from metastases. Many cancers, including breast and head/neck cancers, spread to regional lymph nodes through the lymphatic vessels.

Intravenous administration of chemotherapeutic agents through blood vessels is a common treatment for metastatic lymph nodes. Drugs administered intravenously leak from the capillaries into the interstitium in peripheral tissues and are reabsorbed by the blood vessels and lymphatic vessels.

The lymphatic system is characterized by the preferential uptake of large-sized particles and polymers. Particles with sizes ranging from 10 to 100 nm are easily reabsorbed into the lymphatic system, and particles ≤ 10 nm in size are mainly reabsorbed into blood vessels. The efficiency of reabsorption into the lymphatic system is positively correlated with molecular weight, and in particular, substances with molecular weights > 16,000 are mainly incorporated into the lymphatic system. Therefore, anticancer drugs, which are generally small molecules, are considered difficult to deliver to the lymphatic system and are delivered to a target lymph node inefficiently.

The key principle of a modern drug delivery system (DDS) is the EPR effect (Enhanced Permeability and Retention effect). Unlike normal vascular endothelium, immature tumor blood vessels constructed by cancer tissue have a wide gap of about 200 nm between vascular endothelial cells.

Therefore, particulate preparations and polymeric preparations with controlled particle sizes of about 50 to 100 nm are passively accumulated in cancer tissue. To date, liposome drugs and micellarized drugs have been developed based on the EPR effect, and there have been many reports of their therapeutic actions on solid tumors.

However, research reports on the treatment of lymph node metastasis have been relatively limited compared to studies on the treatment of solid tumors, and this has been attributed to the lack of a disease model useful for studying lymph node metastasis. Under the circumstances, the present inventors succeeded in establishing MXH10/Mo-lpr/lpr lymphadenopathy mice with lymph nodes equivalent in size to those of humans (Figure 1, Non-Patent Literature 1), and using these mice reported that drug delivery methods based on the EPR effect would not be expected to exert a therapeutic effect on the early metastatic lymph node (Non-Patent Literature 2). This is considered to be because blood vessels for supplying oxygen and nutrients necessary for the proliferation of tumor cells invading and engrafting the lymph node are sufficiently present in the normal lymph node, and thus angiogenesis is unlikely to occur at tumor formation in the early stage of lymph node metastasis.

The present invention and colleagues have proposed lymphatic drug delivery as a new treatment for metastatic lymph nodes that does not rely on conventional systemic or oral administration (Non-Patent Literature 3, Non-Patent Literature 4). The concept is to inject the drug locally into the regional lymph node around the primary site to treat or prevent the regional lymph node from discharging metastases downstream, or to administer the drug to the lymph node located upstream of the metastatic lymph node and deliver the drug via lymphatic vessels to the downstream metastatic lymph node.

CITATION LIST

[Non-Patent Literature]

[Non-Patent Literature 1] Shao L, Mori S, Yagishita Y, Okuno T, Hatakeyama Y, Sato T, Kodama T. Lymphatic mapping of mice with systemic lymphoproliferative disorder: usefulness as an inter-lymph node metastasis model of cancer. J Immunol Methods 2013; 389:69-78.
[Non-Patent Literature 2] Mikada M, Sukhbaatar A, Miura Y, Horie S, Sakamoto M, Mori S, Kodama T. Evaluation of the enhanced permeability and retention effect in the early stages of lymph node metastasis. Cancer Sci 2017; 108:846-852.
[Non-Patent Literature 3] Kodama T, Hatakeyama Y, Kato S, Mori S. Visualization of fluid drainage pathways in lymphatic vessels and lymph nodes using a mouse model to test a lymphatic drug delivery system. Biomed Opt Express 2015; 6:124-34.
[Non-Patent Literature 4] Kodama T, Matsuki D, Tada A, Takeda K, Mori S. New concept for the prevention and treatment of metastatic lymph nodes using chemotherapy administered via the lymphatic network. Sci Rep 2016; 6:32506.

## SUMMARY OF THE INVENTION

### TECHNICAL PROBLEM

The present invention aims to provide an optimal intralymphatic preparation containing a drug such as a pharmaceutical substance, a cell, or a nucleic acid for use in the lymphatic drug delivery system.

### SOLUTION TO PROBLEM

As a result of extensive studies on intralymphatic administration preparations used in the lymphatic drug delivery system, the present inventors found that a liquid preparation having a particular osmotic pressure range provided high drug retention in the target lymph node, high delivery to the downstream lymph node, and excellent drug efficacy.
The present invention relates to the following 1) to 11).

1) An intralymphatic administration liquid preparation for delivering a drug to a target lymph node by a lymphatic drug delivery method, the preparation comprising a drug, wherein the osmotic pressure of the liquid ranges from 700 to 2,700 kPa.
2) The preparation according to 1), wherein the osmotic pressure of the liquid is $\geq$ 900 kPa.
3) The preparation according to 1), wherein the osmotic pressure of the liquid is $\leq$ 2,400 kPa.
4) The preparation according to 1), wherein the osmotic pressure of the liquid ranges from 950 to 2,000 kPa.
5) The preparation according to any one of 1) to 4), wherein the viscosity of the liquid is in the range 0.5 to 20 mPa·s.
6) The preparation according to any one of 1) to 4), wherein the viscosity of the liquid ranges from 1.0 to 15 mPa·s.
7) The preparation according to any one of 1) to 6), wherein the preparation comprises a nonionic surfactant.
8) The preparation according to 7), wherein the nonionic surfactant is a polyoxyethylene sorbitan fatty acid ester.
9) The preparation according to 8), wherein the polyoxyethylene sorbitan fatty acid ester is polyoxyethylene sorbitan oleate.
10) The preparation according to any one of 1) to 9), wherein the drug is a pharmaceutically active substance, a nucleic acid molecule container or a cultured cell.
11) The preparation according to any one of 1) to 9), wherein the drug is an anticancer drug.

### Advantageous Effect of the Invention

The present invention provides an intralymphatic preparation for effectively exerting the effects of a drug of interest on a target lymph node using the lymphatic drug delivery system. That is, the lymphatic drug delivery system facilitates adjustment of the delivery rate from the lymph node to the lymph node located downstream of the network and the retention time of pharmaceutically active substances such as a small molecule compound, a polypeptide, an antibody, a nucleic acid, and various cultured cells used in immunotherapy, gene therapy, or regenerative therapy, and to optimize the therapeutic or prophylactic therapeutic effect of the administered drug in the lymph node and the downstream lymph node.

### BRIEF DESCRIPTION OF THE DRAWINGS

Figure 1 shows a diagram of the lymph network in the mouse. The proper axillary lymph node (PALN) and accessory axillary lymph node (AALN) are present in the axillary region. The accessory axillary lymph node and subiliac lymph node (SiLN) are located upstream of the lymphatic networks relative to the proper axillary lymph node. Thus, there is lymphatic flow from the accessory axillary lymph node => proper axillary lymph node and from the subiliac lymph node => proper axillary lymph node.
Figure 2 shows the relationship between polysorbate 80 volume% and viscosity.
Figure 3 shows the kinetics of solution delivery from the subiliac lymph node to the proper axillary lymph node by the lymphatic drug delivery system.
Figure 4 shows the retention properties of solutions that accumulate in the subiliac lymph node and the proper axillary lymph node.
Figure 5 shows body weight changes of mice after the administration of each solution.

Figure 6 shows pathological features of the subiliac lymph node and the proper axillary lymph node on Day 6 from the date of injection.

Figure 7A shows the dynamics of solutions flowing out of the subiliac lymph node (SiLN) to the proper axillary lymph node (PALN) on Day 0.

Figure 7B shows antitumor effects on Day 6 (*in vivo* images).

Figure 8 shows the antitumor effects of solutions with different osmotic pressures on Day 6$^T$ (6 days after the initiation of treatment).

Figure 9 shows the antitumor effects of solutions with different viscosities on Day 6.

Figure 10 shows images of mice on Day 3$^T$ and Day 6$^T$ after the initiation of treatment with the respective solutions.

Figure 11 shows B-mode images of the proper axillary lymph node obtained by high-frequency ultrasound on the day of the experiment.

Figure 12 shows pathological images of the subiliac lymph node and the proper axillary lymph node obtained on Day 6$^T$.

Figure 13 shows mouse body weight changes for each solution.

Figure 14 shows the relationship between glucose volume percent and viscosity.

Figure 15 shows a conceptual diagram of an epirubicin experiment.

Figure 16 shows pathological images on Day 16 after tumor cell inoculation.

Figure 17 shows a conceptual diagram of the nimustine experiment.

Figure 18 shows pathological images on Day 16 after tumor cell inoculation.

Figure 19 shows a conceptual diagram of the methotrexate experiment.

Figure 20 shows pathological images on Day 16 after tumor cell inoculation.

Description of embodiment

In the present invention, "lymphatic drug delivery" denotes a method for administering a drug to a lymph node located upstream of a metastatic lymph node and delivering the drug to a downstream lymph node via a lymphatic vessel.

In the present invention, an "intralymphatic preparation" denotes a liquid preparation administered into a lymph node for the delivery of the drug of interest to a target lymph node by a lymphatic drug delivery system.

In the present invention, "drug" denotes a variety of substances administered into the body of animals, including humans, for the treatment or prophylactic treatment of disease, including pharmaceutically active substances (e.g., small molecule compounds, in particular small molecule organic compounds; proteins or polypeptides (cell growth factors, cell growth inhibitors, neurotrophic factors, enzymes, hormones, cytokines, etc.); polysaccharides; lipids; antibodies; nucleic acids (DNA molecules, RNA molecules, aptamers, etc.); viruses, etc.); as well as structures containing nucleic acid molecules for gene therapy (viruses, virus-like particles, minicircles, plasmids or vectors (naked DNA), liposomes and/or nanoparticles), immunotherapy, or various cultured stem cells used in gene therapy or regenerative medicine (e.g., multipotent stem cells, stem cells, iPS cells) etc.

As used herein, "treatment" refers to the treatment (immediate treatment) of a subject having a disease, and ameliorating or eliminating the condition, or one or more symptoms caused by the condition. By "prophylactic treatment" is meant the treatment of a subject at risk of becoming ill, but who does not currently have the condition or symptoms.

The type of the pharmaceutically active substance is not particularly limited, and may be any of a central nervous system drug, a peripheral nervous system drug, a sensory organ drug, a circulatory organ drug, a respiratory system drug, a digestive organ drug, a urinary organ drug, a hormone drug, an antitumor drug, a radioactive pharmaceutical, and so on, but an antitumor drug (an anticancer drug) is preferable.

The pharmaceutical form of the above pharmaceutically active substance may be a composition, a micellar preparation such as a polymeric micellar medicament, or a liposome preparation.

Examples of suitable drugs are shown below.

(1) One or more members selected from molecular targeting drugs (ibritumomab tiuxetan, imatinib, everolimus, erlotinib, gefitinib, gemtuzumab ozogamicin, sunitinib, cetuximab, sorafenib, dasatinib, tamibarotene, trastuzumab, tretinoin, panitumumab, bevacizumab, bortezomib, lapatinib, and rituximab).

(2) One or more members selected from alkylating agents (ifosfamide, cyclophosphamide, dacarbazine, temozolomide, nimustine, busulfan, melphalan, and ranimustine).

(3) One or more members selected from antimetabolites (enocitabine, capecitabine, carmofur, cladribine, gemcitabine, cytarabine, cytarabine ocfosfate, tegafur, tegafur/uracil, tegafur/gimeracil/oteracil potassium, doxifluridine, nelarabine, hydroxycarbamide, fluorouracil, fludarabine, pemetrexed, pentostatin, mercaptopurine, and methotrexate).

(4) One or more members selected from plant alkaloids (irinotecan, etoposide, eribulin, sobuzoxane, docetaxel, nogitecan, paclitaxel, paclitaxel injection, vinorelbine, vincristine, vindesine, and vinblastine).

(5) One or more members selected from anticancer antibiotics (actinomycin D, aclarubicin, amrubicin, idarubicin, epirubicin, dinostatin stimaramar, daunorubicin, doxorubicin, pirarubicin, bleomycin, peplomycin, mitomycin C, and mitoxantrone).

(6) One or more members selected from the group consisting of platinum preparations (oxaliplatin, carboplatin, cisplatin, and nedaplatin) .

(7) One or more members selected from hormonal agents (anastrozole, exemestane, estramustine, ethinylestradiol, chlormadinone, goserelin, tamoxifen, dexamethasone, toremifene, bicalutamide, flutamide, prednisolone, phosphestrol, mitotane, methyltestosterone, medroxyprogesterone, mepitiostane, leuprorelin and letrozole).

(8) One or more members selected from the group consisting of interferon $\alpha$, interferon $\beta$, interferon $\gamma$, interleukin 2, ubenimex, dried BCG and lentinan.

(9) A micellar preparation of (1) to (8) above.

(10) A liposome preparation of (1) to (8) above.

(11) Various cell suspensions used in immunotherapy, gene therapy, regenerative medicine, and the like.

The intralymphatic preparation of the present invention is preferably an intralymphatic injection dosage form, i.e., an injectable composition. The injectable compositions may be in the form of sterile aqueous or nonaqueous solutions, suspensions, emulsions, gels, or solid compositions for preparation at the time of use.

The intralymphatic preparation of the present invention may be prepared using aqueous diluents, e.g., distilled water for injection, saline, Ringer's solution, phosphate buffer (PBS), or non-aqueous diluents, e.g., vegetable oils, e.g., propylene glycol, polyethylene glycol, olive oil; alcohols, e.g., ethanol; polyoxyethylene sorbitan fatty acid esters, e.g., polysorbate 20, 60, 80, etc., to prepare solutions, suspensions, emulsions of the agent, wherein the osmotic pressure of the liquid ranges from 700 to 2,700 kPa.

The osmotic pressure of the intralymphatic preparation is adjusted in the range 700 to 2,700 kPa, but is preferably $\geq$ 700 kPa, more preferably $\geq$ 800 kPa, more preferably $\geq$ 900 kPa, more preferably $\geq$ 950 kPa, more preferably $\geq$ 1,000 kPa, and preferably $\leq$ 2,700 kPa, preferably $\leq$ 2,400 kPa, more preferably $\leq$ 2,000 kPa, in view of the delivery rate to and retention of the drug in the lymph node located downstream, and potential tissue damage. For example, it is 700 to 2,700 kPa, preferably 900 to 2,700 kPa, more preferably 900 to 2,400 kPa, more preferably 950 to 2,400 kPa, more preferably 950 to 2,000 kPa.

In the present invention, the osmotic pressure, $\pi$, is calculated from the Van't Hoff equation defined by $\pi$ = CRT. In the formula, C denotes the molar concentration (mol/L), R denotes the gas constant (R = 8.31 $\times$ $10^3$ Pa·L/K·mol), and T denotes the absolute temperature (K).

For example, the osmolarities $\pi$ (Pa) of the solutions shown in Table 1 below were obtained by (molar concentration of polysorbate 80 + molar concentration of ethanol) $\times$ 8.31 $\times$ $10^3$ $\times$ (273 + temperature in degrees Celsius).

The intralymphatic preparation of the present invention may have, in terms of the drug effect and the handling of the preparation, a viscosity of $\leq$ 25 mPa·s, more preferably $\leq$ 20 mPa·s, more preferably $\leq$ 15 mPa·s, more preferably $\leq$ 13 mPa·s, and more preferably $\geq$ 0.5 mPa·s, and more preferably $\geq$ 1 mPa·s.

For example, the viscosity range is from 0.5 to 25 mPa·s, preferably from 1.0 to 15 mPa·s, and more preferably from 1.0 to 13 mPa·s.

Viscosity can be measured at 20°C using a vibrating viscometer, e.g., a tuning fork vibratory viscometer (SV-1A, manufactured by A & D, Inc.) as described in the examples below.

The osmotic pressure can be adjusted by using sugars such as glucose, mannitol, sorbitol, sucrose, trehalose, raffinose, and maltose, salts such as sodium chloride and potassium chloride, sugar alcohols or polyhydric alcohols or ethers thereof, nonionic surfactants, and the like. The viscosity can be adjusted by using various hydrophilic polymers generally used as thickeners for injection preparations. Specifically, examples of the hydrophilic polymers include linear polysaccharides such as cellulose, amylose, pectin, gelatin, dextrin, alginate; hydroxyalkyl cellulose, carboxymethyl cellulose, such as cellulose derivatives (methyl cellulose (MC), hydroxypropyl cellulose (HPC) and hydroxypropylmethyl cellulose (HPMC)); non-sulfated glycosaminoglycans such as hyaluronic acid and its salts, desulfated heparin, desulfated chondroitin sulfate, and desulfated dermatan sulfate, etc.; galactomannan (guar gum, tara gum, and locust bean gum, etc.); carbomer; polyacrylic acid; polyvinylpyrrolidone; polyvinyl alcohol; and derivatives and mixtures of polyvinyl acetate.

Among these, it is preferable to use a nonionic diluent capable of adjusting both osmotic pressure and viscosity, for example, sugars such as glucose, mannitol, sorbitol, sucrose, trehalose, raffinose, maltose, but nonionic surfactants are more preferable. Examples of nonionic surfactants include higher alcohol ethylene oxide adducts, alkyl phenol ethylene oxide adducts, fatty acid ethylene oxide adducts, polyhydric alcohol fatty acid ester ethylene oxide adducts, higher alkylamine ethylene oxide adducts, fatty acid amide ethylene oxide adducts, oil/fat ethylene oxide adducts, glycerol fatty acid esters, pentaerythritol fatty acid esters, polyhydric alkyl ethers, alkanolamine fatty acid amides, and among them, sorbitol and a fatty acid ester thereof, polyoxyethylene sorbitan fatty acid ester, polyethylene glycol fatty acid ester, sucrose fatty acid ester, polyoxyethylene castor oil, polyethoxylated hydrogenated castor oil, polyoxyethylene polypropylene glycol copolymers, glycerol fatty acid esters, polyglycerol fatty acid esters, and the like are preferably used.

Examples of the sorbitan fatty acid ester include sorbitan monostearate, sorbitan sesquioleate, sorbitan trioleate, and the like. Examples of the polyoxyethylene sorbitan fatty acid esters include polyoxyethylene sorbitan monolaurate (polysorbate 20, Tween 20), polyoxyethylene sorbitan monostearate (polysorbate 60, Tween 60), polyoxyethylene sorbitan tristearate (polysorbate 65, Tween 65), polyoxyethylene sorbitan oleate (polysorbate 80, Tween 80), and the like. Examples of the polyethylene glycol fatty acid ester include, in particular, polyethylene glycol monolaurate (10 E.O.) or the like. Examples of sucrose fatty acid esters include, particularly, sucrose palmitate esters, sucrose stearate esters, and the like. Particularly preferable examples of the polyoxyethylene castor oil (polyethoxylated castor oil) include polyoxyethylene glycerol trilysinolate 35 and the like. Particularly preferable examples of the polyoxyethylene hardened castor oil (polyethoxylated hydrogenated castor oil) include polyoxyethylene hardened castor oil (50), polyoxyethylene hardened castor oil (60), and the like. Particularly preferable examples of the polyoxyethylene polyoxypropylene glycol copolymer include polyoxyethylene (160), polyoxypropylene (30) glycol, and the like. Preferable examples of the glycerin fatty acid ester include glyceryl monostearate, and the like. Particularly preferable examples of the polyglycerol fatty acid ester include tetraglycerol monostearic acid, decaglycerol monolauric acid, and the like.

Preferable examples of the nonionic surfactant include polyoxyethylene sorbitan fatty acid ester, and more preferably polysorbate 20, polysorbate 60, polysorbate 65, or polysorbate 80, and even more preferably polysorbate 80 (polyoxyethylene sorbitan oleate).

The volume percentage of the nonionic surfactant in the preparation when using a nonionic diluent for the preparation of osmotic pressure is preferably, for example, from 0 to 25% (v/v), more preferably from 8 to 25% (v/v), more preferably from 10 to 23% (v/v), and even more preferably from 15 to 20% (v/v).

The intralymphatic preparation of the present invention may be prepared according to conventional methods in the same manner as known injection preparations, by incorporating adjuvants (diluent) such as surfactants, preservatives (stabilizers), analgesics, topical anesthetics and pH-adjusting agents, preservatives, wetting agents, emulsifying agents, dispersing agents, stabilizing agents and dissolving aids, as appropriate, with the above drugs and osmotic pressure preparations to the extent that they do not interfere with the effects of the present invention.

Examples of the preservatives include alkyl parabens such as methyl paraben and propyl paraben, examples of the analgesics include benzyl alcohol, examples of the topical anesthetics include xylocaine hydrochloride and chlorobutanol, and examples of the pH adjusting agents include hydrochloric acid, acetic acid, sodium hydroxide, and various buffers.

Examples of the surfactants, dispersing agents and emulsifying agents include the above nonionic surfactants, polyethylene glycol, carboxymethylcellulose, sodium alginate, and the like.

Examples of solubilizing agents include sodium salicylate, poloxamer, and sodium acetate; examples of the preservative include methyl paraben, propyl paraben, benzyl alcohol, chlorobutanol, sodium benzoate, and phenol; and examples of the stabilizing agent include albumin such as human serum albumin and bovine serum albumin.

The intralymphatic preparation of the present invention thus prepared may be administered locally into the lymph nodes of patients. Here, the lymph node to be administered may be the lymph node itself intended for therapeutic or prophylactic treatment, or may be a lymph node located upstream of the lymphatic network to which the lymph node belongs. Specifically, for example, a sentinel lymph node in which tumor cells first migrate from the primary tumor, a lymph node located downstream of the sentinel lymph node (secondary lymph node), a lymph node located upstream of the regional lymph node around the primary tumor, and a lymph node located upstream of the lymphatic network to which the regional lymph node belongs. Here, the target lymph node may or may not contain cancer cells. For example, before lymph node dissection, the lymph node in the dissection area (upstream lymph node) may be treated prophylactically by administering an intralymphatic preparation and delivering an anticancer drug via the lymphatic network to a lymph node outside the dissection area (downstream lymph node); this treatment is then followed by dissection.

The modes of administration of the intralymphatic preparation of the present invention to the lymph node are not limited as long as the preparation can be injected into the lymph node, and the preparation may be administered by injection into the lymph node after the lymph node has been exposed by incising the patient's skin, or may be administered by injection from above the patient's skin to a site where the lymph node appears to be located.

EXAMPLES

In the following, the present invention will be explained in more detail on the basis of examples, but it is not limited thereto.

REFERENCE EXAMPLE 1: Measurement of the viscosity of indocyanine green (ICG)-containing solution

1. Materials and methods

1) Preparation of the solution

Polysorbate 80 (polysorbate 80, Nippon Oil Co., Ltd.), distilled water, and indocyanine green solution (Indocyanine Green: ICG, Daiichi Sankyo Co., Ltd.) were mixed to prepare solutions of differing viscosities (Table 1).
The viscosities of the solutions in Table 1 were determined by a tuning fork vibrating viscometer (SV-1A: viscosity measuring range: 0.3 to 10,000 mPa·s, SV-1H: viscosity measuring range: 0.3 to 1,000 mPa·s, A&D Co., Ltd.), and were measured at room temperature (20°C).
In Table 1, the ratio to the osmotic pressure of blood is a ratio obtained by converting the osmotic pressure [kPa] of each solution to mOsm/kg from the relationship of 1 mOsm/kg = 2269.68 Pa and dividing this by the osmotic pressure of blood of 290 mOsm/kg.

2. Results

1) Relationship between polysorbate 80 vol% and viscosity

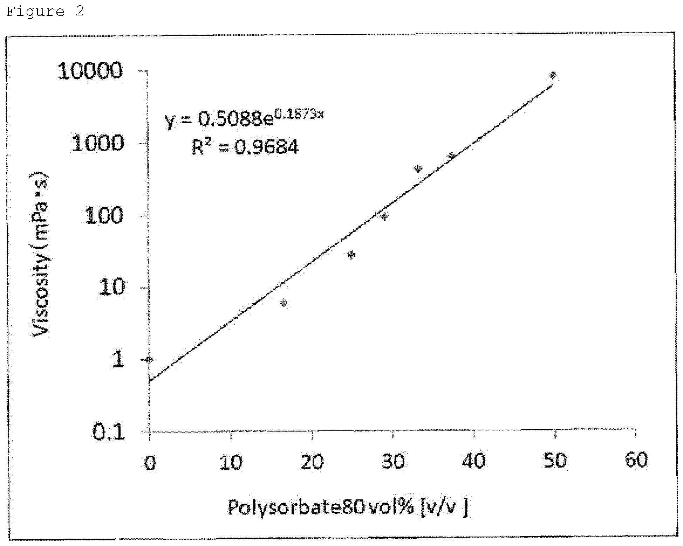

Figure 2 shows the relationship between polysorbate 80 vol% and viscosity. The higher the polysorbate 80 content, the more viscosity tends to increase exponentially.
Assuming polysorbate 80 vol% (x) and viscosity (y), the following relation (equation 1) was obtained.

[equation 1]

$$y = 0.5088e^{0.1873x}$$

The osmotic pressures in Table 1 were obtained from the following Van't Hoff equation (equation 2).

[Equation 2]

$$\Pi = CRT$$

$\pi$: osmotic pressure [Pa]

$$C = n/V$$

C: molar concentration [mol/L]
n: total molar number in solution [mol]
V: volume of solution [L]
R: gas constant (8.31 x 103) [Pa·L/(K·mol)]
T: absolute temperature [K]

Table 1

|  | Solution A | Solution B | Solution C | Solution D |
|---|---|---|---|---|
| Polysorbate 80 ($\mu$L) | 0 | 167 | 333 | 500 |
| Distilled water ($\mu$L) | 600 | 400 | 200 | 0 |
| ICG-diluted standard liquid ($\mu$L) | 400 | 400 | 400 | 400 |
| 100% ethanol ($\mu$L) | 0 | 33 | 67 | 100 |
| Total volume ($\mu$L) | 1000 | 1000 | 1000 | 1000 |
| Polysorbate vol% (v/v) | 0.00 | 16.67 | 33.33 | 50.00 |
| Viscosity (mPa · s) | 1.01 | 6.01 | 427 | 8020 |

(continued)

|  | Solution A | Solution B | Solution C | Solution D |
| --- | --- | --- | --- | --- |
| Osmotic pressure $\pi$ (kPa) | 0 | 1740 | 3481 | 5221 |
| Ratio to osmotic pressure of blood | 0.0 | 2.6 | 5.3 | 7.9 |

EXAMPLE 1: Pharmacokinetics of Lymphatic Drug Delivery with Solutions of Varying Osmotic Pressures

1. Materials and methods

1) Preparation of solution

Four solutions (Solution A, B, C, and D) shown in Table 1 were prepared. The osmotic pressure and viscosity of each solution were:

Solution A: Osmotic pressure = 0 kPa, viscosity = 1.01 mPa·s
Solution B: Osmotic pressure = 1,740 kPa, viscosity = 6.01 mPa·s
Solution C: Osmotic pressure = 3,481 kPa, viscosity = 427 mPa·s
Solution D: Osmotic pressure = 5,221 kPa, viscosity = 8020 mPa·s

2) Visualization of solutions with different osmotic pressures delivered by a drug delivery system via lymphatic vessels.

Solutions were injected into the subiliac lymph node (SiLN) at a dose rate of 10 $\mu$L/min in a volume of 200 $\mu$L, and delivery to and retention in the proper axillary lymph node (PALN) through the lymphatic duct were recognized in the fluorescence mode of the bioluminescence imaging system (manufactured by IVIS, PerkinElmer) (Excitation filter: 745 nm, Emission filter: ICG).

The observation timings were as follows: immediately before, immediately after, 6 hours after, 1 day after, 2 days after, 3 days after, 4 days after, 5 days after, 6 days after, 7 days after, 14 days after, 21 days after, 28 days after, 35 days after, 42 days after, and 49 days after injection of the indocyanine green solution into the subiliac lymph node.

3) Retention properties of the accumulated solutions in the subiliac lymph node and the proper axillary lymph node.

The relationships between the fluorescence values of the subiliac lymph node and the proper axillary lymph node obtained at the timings of 2) above and the measurement time were plotted.

4) Body weight changes associated with indocyanine green solution infusion using the lymphatic drug delivery system.

Body weight measurements were recorded immediately before, and 1 day, 2 days, 3 days, 4 days, 5 days, 6 days, 7 days, 14 days, 21 days, 28 days, and 35 days after injection of indocyanine green solution into the subiliac lymph node. Each round of body weight measurements was expressed as the average $\pm$ standard error (mean $\pm$ SE).

2. Experimental results

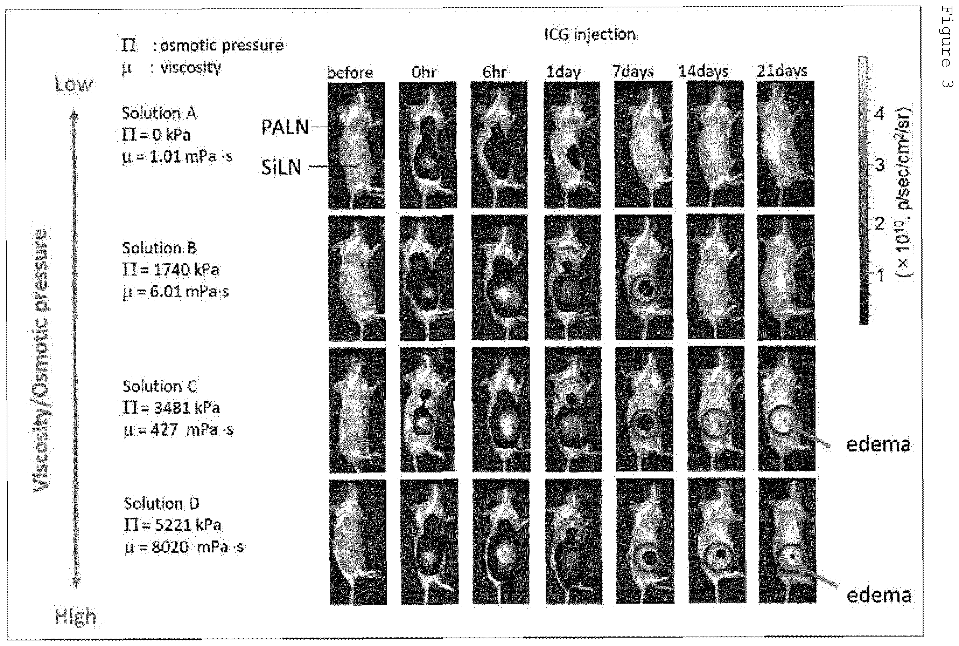

1) Kinetics of Solution Delivery from the Subiliac Lymph Node to the Proper Axillary Lymph Node by the Lymphatic Drug Delivery System Figure 3 shows the kinetics of solution delivery from the subiliac lymph node to the proper axillary lymph node produced by the lymphatic drug delivery system.

Immediately after administration, flow from the subiliac lymph node to the proper axillary lymph node was confirmed for all solutions (0 h).

One day after injection, retention in the proper axillary lymph node was confirmed for Solution B, C, and D (circled areas), but not for Solution A.

On the seventh day after injection, retention of Solution B, C, and D in the subiliac lymph node (circled area) was confirmed, but retention of solution A was not confirmed.

On the 14th day after injection, retention of Solution C and D in the subiliac lymph node was confirmed (circled area), but retention of Solution A and B was not confirmed.

On the 21st day after injection, retention of Solution D was confirmed in the subiliac lymph node (circled area), but retention of solutions A, B, and C was not confirmed.

For Solution D, considerable force was required to place Solution D into the injection container and push it out through the injection needle, which was not suitable for practical use.

Use of Solution C and D resulted in an edematous subiliac lymph node (arrows).

2) Retention properties of the accumulated solutions in the subiliac lymph node and the proper axillary lymph node

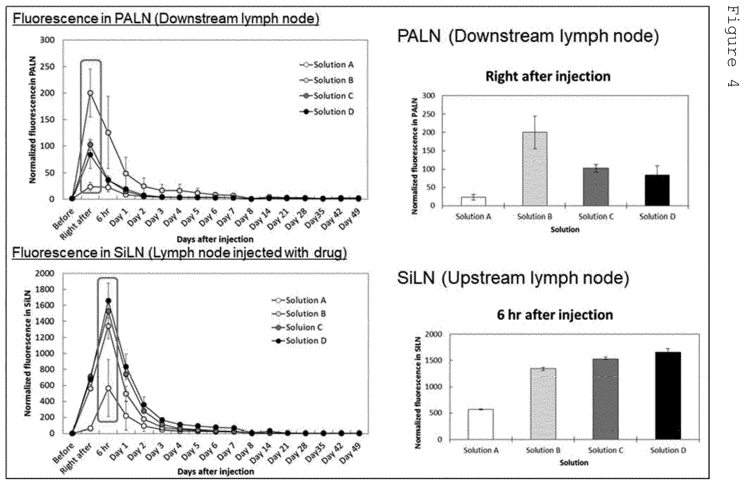

Figure 4 demonstrates the retention characteristics of solutions accumulated in the subiliac lymph node and the proper axillary lymph node shown in Figure 3. Immediately after administration, it was confirmed that the retention of Solution B was high in the proper axillary lymph node, and the retention of Solution D was high in the subiliac lymph node.

3) Body weight changes for each solution

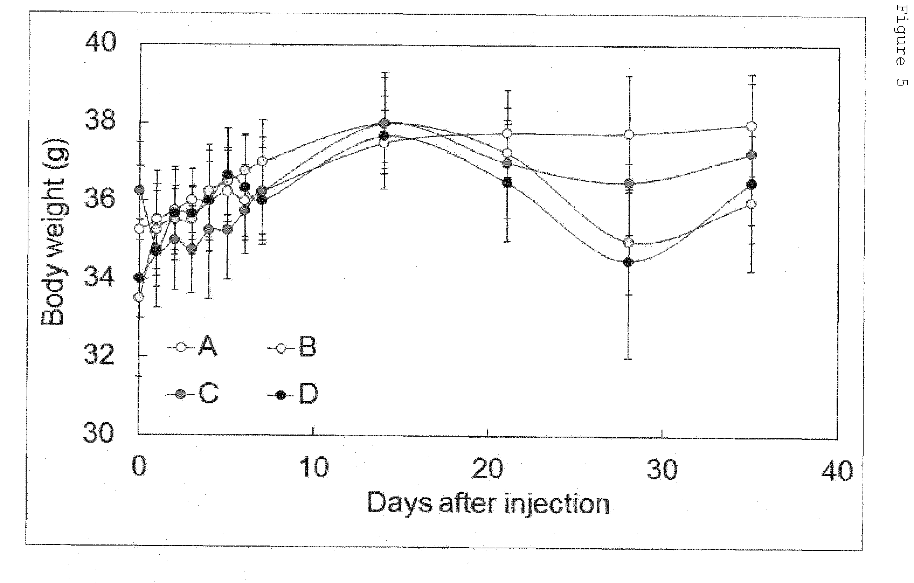

Figure 5 shows the body weight changes of the mice after the administration of each solution. Each data value is expressed as the mean $\pm$ standard error. Injection of Solution A, B, C, or D into the subiliac lymph node did not produce significant weight loss as a side effect.

4) Appropriate range of osmotic pressure and viscosity

From the above, it can be judged that an appropriate range of osmotic pressure and viscosity is $\leq 3,481$ kPa for the upper limit of the osmotic pressure and $\leq 427$ mPa $\times$ s for the upper limit of viscosity.

EXAMPLE 2: Pathological Evaluations for Solutions with Different Osmotic Pressures

1. Materials and methods

1) Solution preparation

The solutions shown in Table 2 were used: $\pi$ 758, $\pi$ 408, $\pi$ 515, $\pi$ 556, $\pi$ 2610, and $\pi$ 2773. As for the viscosity of $\pi$ 758, the viscosity of physiological saline was judged to be equivalent to that of water, and was obtained from reference literature (Science Data, National Astronomical Observatory, 1997, Maruzen Co., Ltd.). The viscosities of the solutions $\pi$ 758, $\pi$ 408, $\pi$ 515, $\pi$ 556, $\pi$ 2,610, and $\pi$ 2,773 were measured by a tuning fork vibrating viscometer (SV-1A: viscosity measurement range: 0.3 to 10,000 mPa·s, SV-1H: viscosity measurement range: 0.3 to 1,000 mPa·s, A&D Co., Ltd.) at room temperature (20°C).

The osmotic pressure was obtained from equation 2 above in the same manner as described previously (*vide supra*).

In Table 2, the ratio to the osmotic pressure of blood is the ratio obtained by converting the osmotic pressure [kPa] of each solution into units of mOsm/Kg from the relationship of 1 mOsm/Kg = 2269.68 Pa, and dividing the value by the osmotic pressure of blood (290 mOsm/kg).

Table 2

| Solution | Π 758 | Π 408 | Π 515 | Π 556 | Π 2,610 | Π 2,773 |
|---|---|---|---|---|---|---|
| Polysorbate 80 ($\mu$L) | 0 | 10 | 20 | 24 | 220 | 239 |
| 100% ethanol ($\mu$L) | 0 | 2 | 4 | 4.8 | 44 | 48 |
| Distilled water ($\mu$L) | 0 | 588 | 576 | 571.2 | 336 | 314 |
| Saline ($\mu$L) | 1,000 | 400 | 400 | 400 | 400 | 400 |
| Total ($\mu$L) | 1,000 | 1,000 | 1,000 | 1,000 | 1,000 | 1,001 |
| | | | | | | |
| Polysorbate 80 (%) | 0.0 | 1.0 | 2.0 | 2.4 | 22.0 | 23.9 |
| Viscosity (mPa ·s) | 1.04 | 1.0 | 0.93 | 1.05 | 13.9 | 18.9 |
| Osmotic pressure (kPa) | 758 | 408 | 515 | 556 | 2,610 | 2,773 |
| Ratio to osmotic pressure of blood | 1.2 | 0.6 | 0.8 | 0.8 | 4.0 | 4.2 |

2) Mice

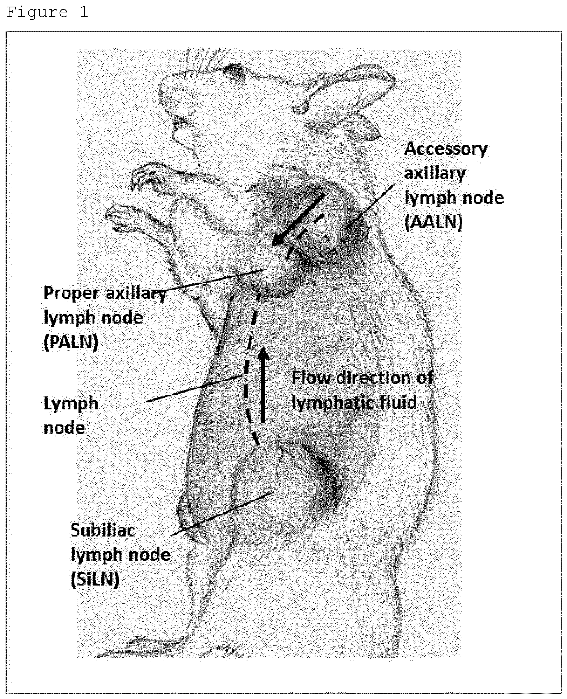

MXH10/Mo-lpr/lpr mice (14-18 weeks old) shown in Figure 1 (non literature 1 above) were used.

3) Injection of the solution

The solutions were injected into the subiliac lymph node (SiLN) at a rate of 10 μL/min in a volume of 200 μL and delivered via the lymphatics to the proper axillary lymph node (PALN). The day of injection was defined as Day 0.

4) Histological analysis

On the sixth day after injection (Day 6), the subiliac lymph node and the proper axillary lymph node were removed, immersed in 10% formalin solution, left for 4 days, and then embedded in paraffin to prepare blocks. Paraffin blocks were sliced to a thickness of 3 μm using a microtome (REM-700, Yamato Kohki, Saitama, Japan) and placed onto glass slides (Superfrost, Matsunami, Osaka, Japan). Subsequently, they were placed on a paraffin stretcher (Thermo Fisher Scientific, Waltham, MA, US) overnight and fully extended. The sections were stained with hematoxylin and eosin (HE) using a HE Fully Automated Staining System (Ventana Symphony, Ventana Medical Systems, Inc., Tucson, AZ, US).
Observation was performed by bright-field microscopy using an optical microscope (BX51, manufactured by Olympus).

2. Results

1) Histological analysis

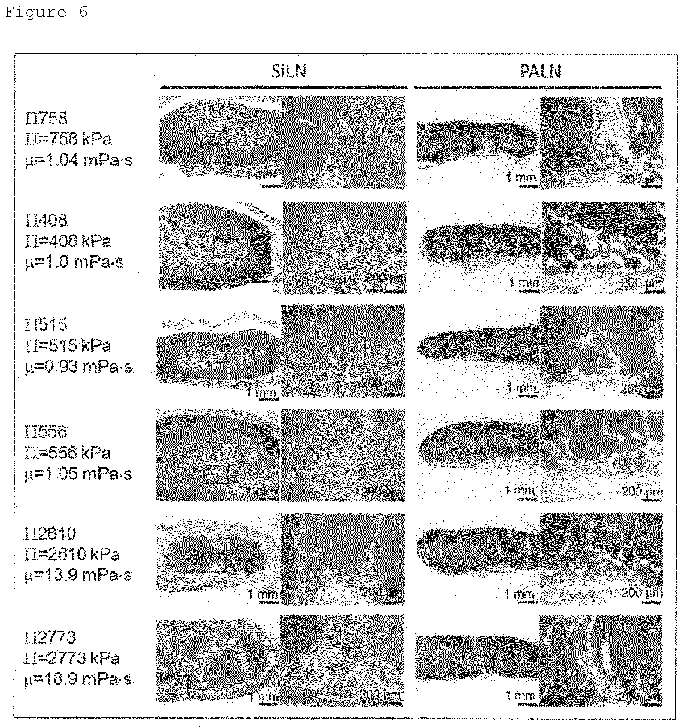

Figure 6 shows pathological images of the subiliac lymph node and the proper axillary lymph node on the 6th day (Day 6) after the injection of solutions. Solutions are $\pi$756, $\pi$408, $\pi$515, $\pi$556, $\pi$2,610 and $\pi$2,773. The magnifications are 2-fold and 10-fold. The area surrounded by □ on the 2-fold image is enlarged to a 10-fold image. In the image, T denotes a tumor region and N denotes a necrotic region.

(A) Π 758

SiLN

No significant pathological changes were recognized in the lymph node.

PALN

No significant pathological changes were recognized in the lymph node.

(B) Π 408

SiLN

Slight enlargement of the lymph node medullary sinus and slight edema of the lymph node medulla were recognized, but no obvious organic changes were recognized in the lymph node.

PALN

Dilatation was recognized in the medullary sinus throughout the lymph node, and edema was recognized outside the lymph node. However, necrotic foci and fibrosis were not recognized. The results seemed to reflect reversible changes.

(C) Π 515

SiLN

Mild enlargement of the lymph node medullary sinus and mild edema of the lymph node medulla were recognized, but no obvious organic changes were detected in the lymph node.

PALN

Mild enlargement of the lymph node medullary sinus and mild edema of the lymph node medulla were recognized, but no obvious organic changes were detected in the lymph node.

(D) Π 556

SiLN

Slight enlargement of the sinuses of the lymph node and edema of the lymph node medulla were recognized without obvious organic changes in the lymph node.

PALN

Dilatation was recognized in the medullary sinus throughout the lymph node, and edema was recognized outside the lymph node. Necrotic foci and fibrosis, however, were not detected, and the changes appeared to be reversible.

(E) Π 2610

SiLN

Slight enlargement of the medullary sinus of the lymph node was recognized.

PALN

Dilatation of the medullary sinus was recognized throughout the lymph node, and edema was detected outside the lymph node. Necrotic foci and fibrosis, however, were not recognized, and the changes appeared to be reversible.

(F) Π 2773

SiLN

Drug delivery via efferent lymphatic vessels produced extensive necrosis in the majority of lymph nodes and necrosis in the efferent lymphatic vessel base.

PALN

Slight enlargement of the lymph node medullary sinus and slight edema of the lymph node medulla were recognized, but no obvious organic changes were detected in the lymph node.
From the above results, it may be considered that a solution having an osmotic pressure of 2,773 kPa and a viscosity of 18.9 mPa·s fails to achieve drug delivery to the downstream lymph node and is not suitable for lymphatic drug delivery.

EXAMPLE 3: Study of the Effective Range of Osmotic Pressures for a Cisplatin-Containing Solution

1. Materials and methods

1) Preparation of solution

Table 3 shows the compositions of the solutions containing cisplatin.
The solutions were: solution I, solution I', solution II, solution II', solution III, solution III', solution IV, solution IV', solution V. The working solution of cisplatin was prepared using saline. Mice were administered a cisplatin dose of 5 mg/kg of body weight.
The viscosities shown in Table 3 were calculated from equation 1.
The osmotic pressures shown in Table 3 were determined from equation 2.
In Table 3, the ratio to the osmotic pressure of blood was obtained by converting the osmotic pressure [kPa] of each solution into units of mOsm/Kg from the relationship of 1 mOsm/Kg = 2,269.68 Pa, and dividing this by the osmotic pressure of blood (290 mOsm/kg).

Table 3

|  | Solution I | Solution I' | Solution II | Solution II' | Solution III | Solution III' | Solution IV | Solution IV' | Solution V |
|---|---|---|---|---|---|---|---|---|---|
| Polysorbate 80 (μL) | 0 | 42 | 83 | 125 | 167 | 208 | 250 | 292 | 333 |
| 100% ethanol (μL) | 0 | 8 | 17 | 25 | 33 | 42 | 50 | 58 | 67 |
| Distilled water (μL) | 600 | 550 | 500 | 450 | 400 | 350 | 300 | 250 | 200 |
| 500 μg/mL ICG (μL) | 200 | 200 | 200 | 200 | 200 | 200 | 200 | 200 | 200 |
| 5 mg/kg CDDP solution (μL) | 200 | 200 | 200 | 200 | 200 | 200 | 200 | 200 | 200 |
| Total (μL) | 1,000 | 1,000 | 1,000 | 1,000 | 1,000 | 1,000 | 1,000 | 1,000 | 1,000 |
| Vol% of polysorbate 80 (v/v) | 0.0 | 4.2 | 8.3 | 12.5 | 16.7 | 20.8 | 25.0 | 29.2 | 33.3 |
| Viscosity (mPa·s) | 0.5 | 1.1 | 2.4 | 5.3 | 11.5 | 25.2 | 55.0 | 120.0 | 261.8 |
| Osmotic pressure Π (kPa) | 152 | 588 | 1,024 | 1,459 | 1,897 | 2,331 | 2,768 | 3,200 | 3,641 |
| Ratio to osmotic pressure of blood | 0.2 | 0.9 | 1.6 | 2.2 | 2.9 | 3.5 | 4.2 | 4.9 | 5.5 |

2) Tumor cells

KM-Luc/GFP malignant fibrous histiocytoma-like cells expressing the luciferase gene were used (Li L, Mori S, Sakamoto M, Takahashi S, Kodama T. Mouse model of lymph node metastasis via afferent lymphatic vessels for development of imaging modalities. PLoS One 2013; 8:e55797) . Cells were cultured in 10% fetal bovine serum (FBS; Sigma-Aldrich, St Louis, Mo., US), 1% L-glutamine-penicillin-streptomycin (Sigma-Aldrich) and Dulbecco's modified Eagle's medium (DMEM, Sigma-Aldrich) containing 0.5% G418 (Sigma-Aldrich). The culture conditions were 37°C and 5% $CO_2$.

3) Cell inoculation

Cell solutions ($4 \times 10^5$ cells/mL) were seeded into the proper axillary lymph node in 40 µL aliquots. This day was defined as Day -$3^T$.

4) Drug administration

Three days after cell inoculation, 200 µL of cisplatin solution was administered to the subiliac lymph node at a rate of 10 µL/min and delivered to the proper axillary lymph node.

5) Confirmation of tumor growth using a bioluminescence imaging system

A bioluminescence imaging system (manufactured by IVIS, PerkinElmer) was used to evaluate tumor growth within the proper axillary lymph node. Under anesthesia, 10 µL/g of luciferin adjusted to 15 mg/mL was injected into the peritoneal cavity of each mouse in accordance with its body weight. Ten minutes after luciferin administration, bioluminescence intensities were measured using IVIS. Measurement data were calculated as luminescence per unit time in the proper axillary lymph node using dedicated analysis software. The bioemissive intensity was measured on the day that treatment was initiated (Day 0), on the third day (Day $3^T$) and on the sixth day (Day $6^T$).

6) Ultrasound evaluation of the proper axillary lymph node

B-mode images of the proper axillary lymph node were obtained using a high-frequency ultrasound diagnostic instrument for small animals (center frequency: 25 MHz, spatial resolution: 70 µm, azimuthal resolution: 140 mm, VEVO770, VisualSonics). Tumors were measured on the day of inoculation (Day -$3^T$), on the day of commencement of treatment (Day $0^T$), on the third day (Day $3^T$) and on the sixth day (Day $6^T$).

7) Lymph node volume measurement

Mice were weighed on the day of tumor inoculation (Day -$3^T$), on the day that treatment was started (Day $0^T$), on the third day (Day $3^T$), and on the sixth day (Day $6^T$).

8) Histological analysis

The subiliac lymph node and the proper axillary lymph node were removed 6 days after the initiation of treatment (day $6^T$), and the pathology was analyzed after hematoxylin-eosin staining.

2. Experimental results

1) Kinetics of the solution

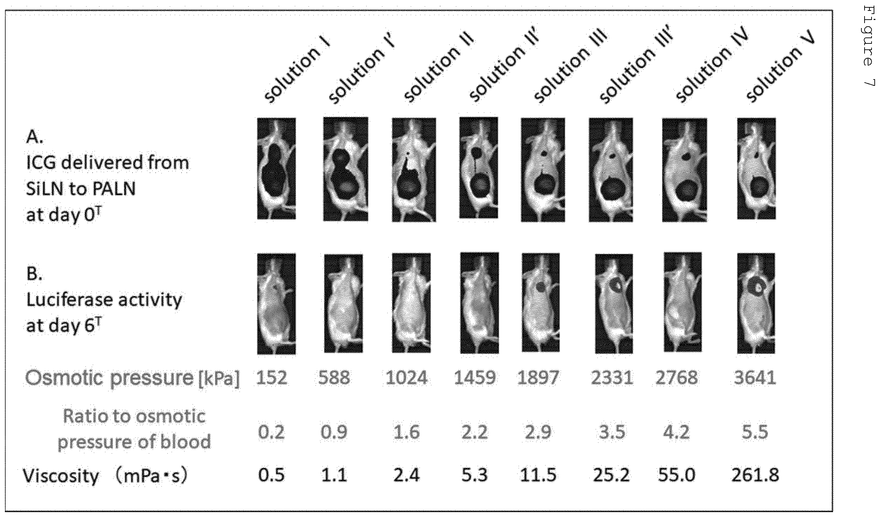

Figure 7A shows *in vivo* images of the kinetics of solutions flowing from the subiliac lymph node (SiLN) to the proper axillary lymph node (PALN) on Day $0^T$ for solution I, solution I', solution II, solution II', solution III, solution III', solution IV, and solution V.

Figure 7B shows *in vivo* images to assess the antitumor effects on Day $6^T$. The ratio to blood osmotic pressure denotes the value of osmotic pressure [kPa] of each solution converted to units of mOsm/Kg from the relationship '1 mOsm/Kg = 2,269.68 Pa' and then divided by the osmotic pressure of blood (290 mOsm/kg).

2) Influence of osmotic pressure on the antitumor effect

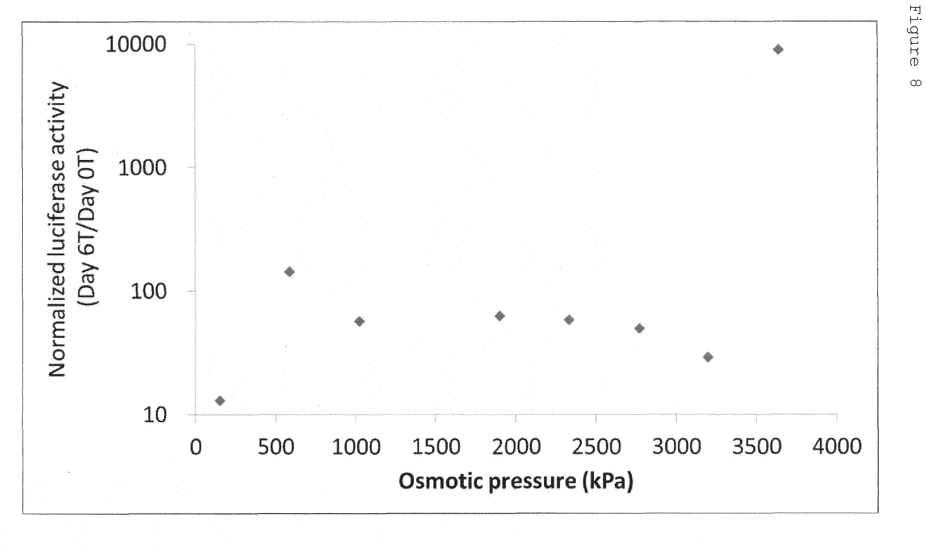

Figure 8 shows the antitumor effects of the solutions with the respective osmotic pressures on Day 6. The vertical axis represents dimensionless luciferase activity values. A dimensionless quantity of luciferase activity (Day $6^T$)/luciferase activity (Day $0^T$) is introduced. As a result, in the evaluation of the antitumor effect using luciferase activity as an index, the antitumor effect in the proper axillary lymph node was confirmed up to an osmolarity of $\pi$ = 3,200 kPa.

3) Influence of viscosity on the antitumor effect

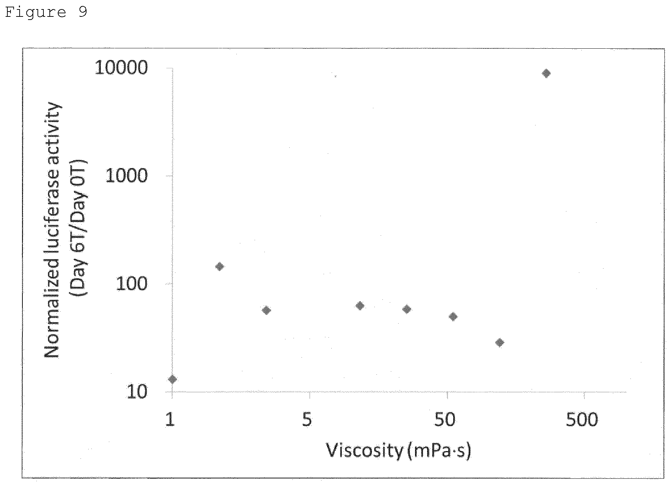

Figure 9 shows the antitumor effects of the solutions with the respective viscosities on Day 6.
As a result, in the evaluation of the antitumor effect using luciferase activity as an index, the antitumor effect in the proper axillary lymph node was confirmed up to a viscosity $\mu$ = 120 mPa·s.

4) Influence of osmotic pressure and viscosity on edema in the macroscopic field of view

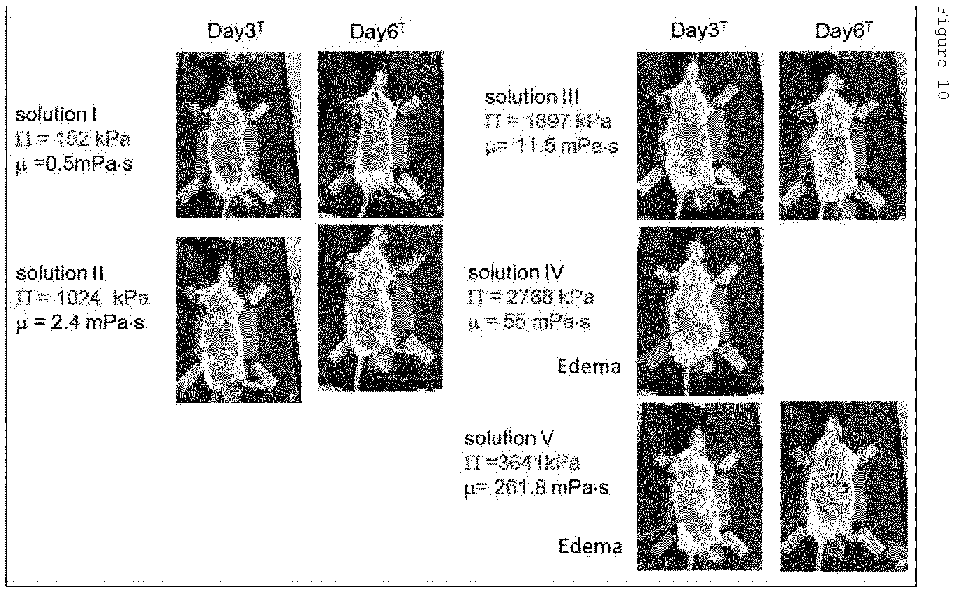

Figure 10 shows images of mice on Day $3^T$ and Day $6^T$ after the start of treatment for all solutions. On Day $3^T$, edema was confirmed for solutions IV (osmotic pressure $\pi$ = 2768 kPa, viscosity $\mu$ = 55 mPa·s) and V (osmotic pressure $\pi$ = 3641 kPa, viscosity $\mu$ = 261.5 mPa ·s).

5) Evaluation of edema by high-frequency ultrasound imaging

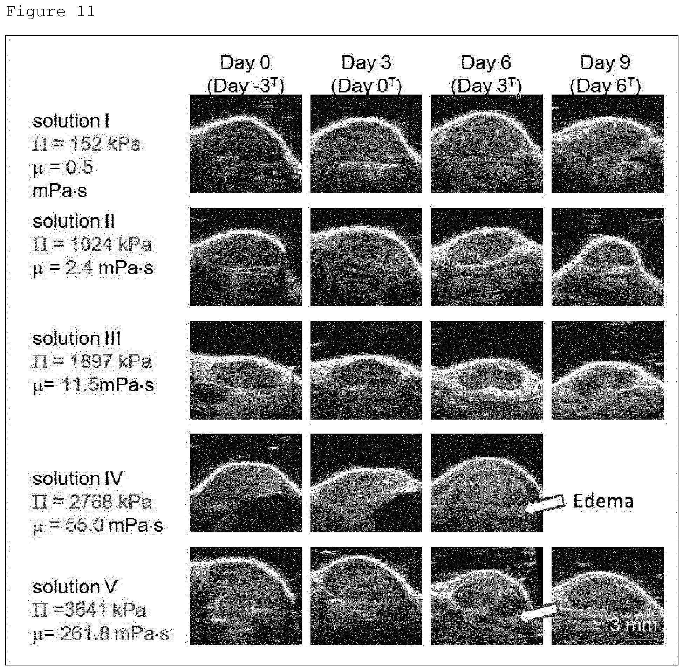

Figure 11 shows B-mode images of the proper axillary lymph node obtained by high-frequency ultrasound on the experimental day. On Day $6^T$, edema was confirmed for solutions IV (osmotic pressure $\pi$ = 2,768 kPa, viscosity $\mu$ = 55.0 mPa·s) and V (osmotic pressure $\pi$ = 3,641 kPa, viscosity $\mu$ = 261.8 mPa·s).

6) Histological analysis

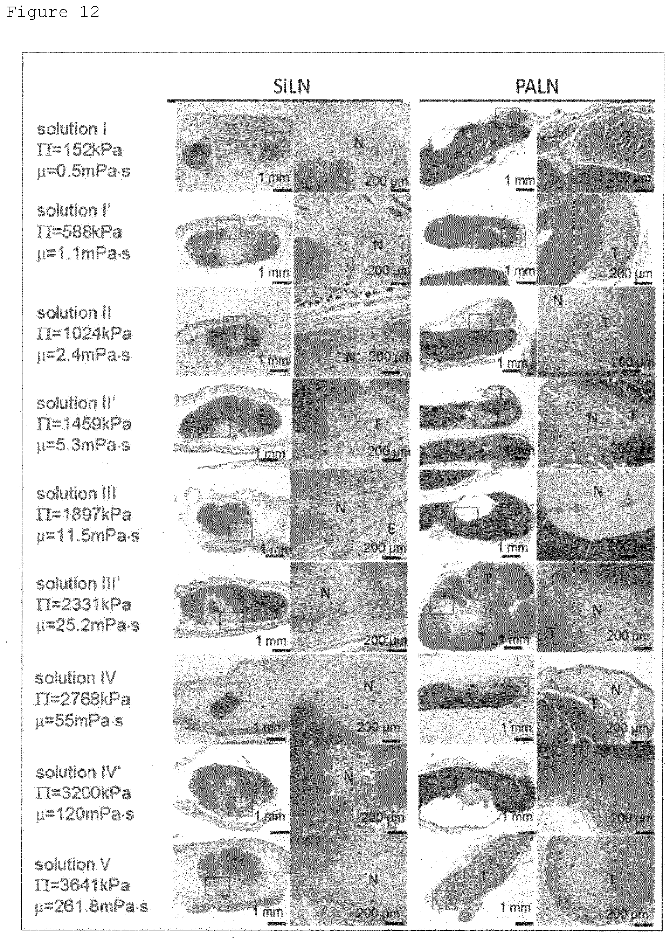

Figure 12 shows the pathology of the subiliac lymph node and the proper axillary lymph node on Day $6^T$ for solution I, solution I', solution II, solution II', solution III, solution III', solution IV, solution IV' and solution V. The magnifications are 2-fold and 10-fold. The area surrounded by □ in the 2-fold image is enlarged to a 10-fold image. In the image, T denotes a tumor region, N denotes a necrotic region, and E denotes an edematous region.

(A) Solution I

SiLN

Most of the existing lymph nodes became necrotic, and the necrosis extended to the surrounding tissues. This result indicates that the anticancer drug had diffused into the surrounding tissues. It is an unexpected finding given that little drug delivery from the efferent lymphatic vessels to the PALN was expected.

PALN

Some regions of tumor tissue in the marginal sinuses of the lymph node became necrotic, while other regions of the marginal sinuses showed little inhibition of tumor growth. The structure of the lymph node parenchyma was preserved. It seems that the anticancer drug flowed into part of the marginal sinus of the lymph node but did not reach the whole region.

(B) Solution I'

SiLN

The formation of extensive necrotic foci, which may have been caused by the injection of the anticancer drug, was recognized in the center of the lymph node, and extended to the marginal sinus. This finding indicates that drug delivery from the efferent lymphatic vessels to the PALN was almost impossible.

PALN

Tumor infiltration and proliferation were recognized, which seemed to have grown from the marginal sinus of the lymph node. It appears that hardly any drug delivery was achieved.

(C) Solution II

SiLN

Though necrotic foci likely formed by the infusion of the anticancer drug were recognized in the center of the lymph node, the tissue of the lymph node marginal sinus was preserved, and drug delivery to the PALN from the efferent lymphatic vessel was expected to occur.

PALN

Tumor cell proliferation was recognized in the marginal sinus of the lymph node but was accompanied by extensive necrosis of the tumor tissue. This action is expected to have resulted from lymphatic delivery of the drug from the SiLN.

(D) Solution II'

SiLN

Though edematous and localized necrosis were recognized in the region corresponding to the hilum of the lymph node, the lymph node structure was conserved, and drug delivery to the PALN from the efferent lymphatic vessel was expected to occur.

PALN

Tumor cell proliferation was seen in the marginal sinus and parenchymal equivalent of the lymph node, but extensive necrosis of the tumor tissue was also noted. It may be considered to have resulted from the effects of lymphatic drug delivery from the SiLN.

(E) Solution III

SiLN

Though the necrotic lesion was limited to one part of the lymph node, the structure of the lymph node was preserved, and drug delivery to the PALN from the efferent lymphatic vessel was expected to occur.

PALN

Tumor cells grew in the marginal sinus of the lymph node and the parenchymal equivalent of the lymph node, and extensive necrotic foci were recognized. No tumor cells remained due to the marked antitumor effect produced by lymphatic drug delivery from the SiLN.

(F) Solution III'

SiLN

Necrotic tissue was recognized in the lymph node, and the marginal sinus was also partially necrotic.

PALN

The proliferation of tumor cells was identified in the lymph node marginal sinus and lymph node parenchyma, and some tumor tissue was necrotic. Although localized, antitumor effects of drug delivered lymphatically from the SiLN were evident. Because of the promising results of lymphatic drug delivery using this solution, it is believed that the desired antitumor effect could be obtained, for example, by increasing the concentration of cisplatin administered.

(G) Solution IV

SiLN

Extensive necrosis of the existing lymph node and marked edema of surrounding tissues occurred. This finding indicates that drug delivery from the efferent lymphatic vessels to the PALN was not expected.

PALN

Tumor with areas of necrosis extended from the marginal sinus of the lymph node. It appears that a sufficient amount of anticancer drug had not been delivered.

(H) Solution IV'

SiLN

Necrotic tissue was recognized in the lymph node region equivalent to the hilum, and edema affected the surrounding tissue. Adequate drug delivery to the PALN from the efferent lymphatic vessels was not expected.

PALN

An extensive area of the lymph node had been replaced by tumor, and very little delivery of anticancer agent appears to have been achieved.

(I) Solution V

SiLN

The area of the lymph node hilum was extensively necrotic, and the surrounding tissue exhibited marked edema, a finding suggesting that little drug delivery from the efferent lymphatic vessels to the PALN was expected.

PALN

All regions of the lymph node had been replaced by tumor. Little anticancer drug appears to have been delivered.
From the above observations, solution II ~ solution III' were the most effective solutions for the lymphatic delivery of a solution containing cisplatin. Based on the pathology results, it may be considered that an osmolarity of ≤ 588kPa or > 2,768 kPa is not suitable for a preparation intended for lymphatic drug delivery.

7) Body weight changes

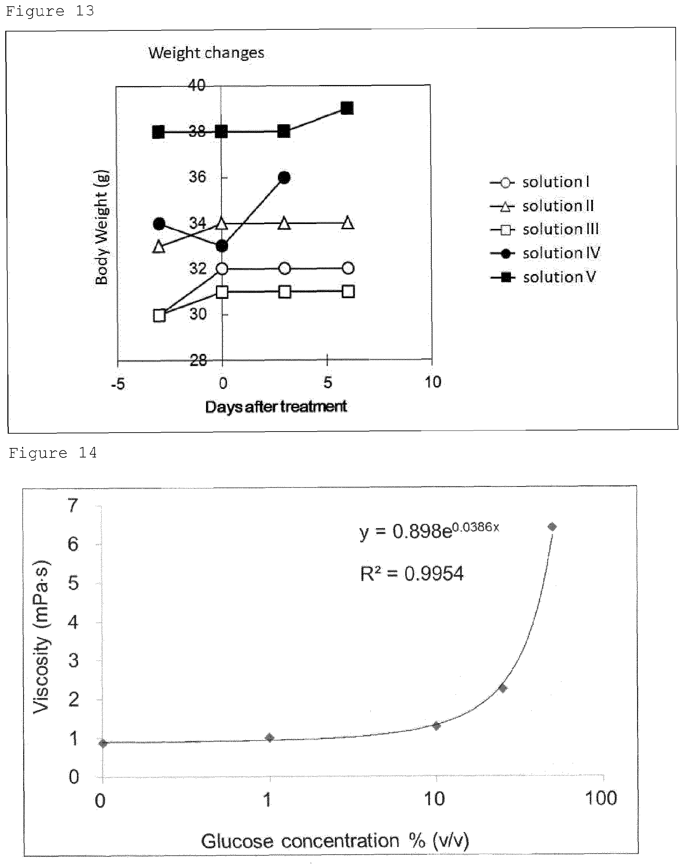

Figure 13 shows the body weight changes of the mice for each solution. No clear weight loss was found for all solutions tested (solution I, II, III, IV, and V).

REFERENCE EXAMPLE 2: Measurement of the Viscosity of Glucose-Containing Solution

1. Materials and methods

1) Preparation of the solution

Glucose (Otsuka, 50% glucose) was diluted, and solutions of varying viscosities (0.1-50 v/v% aqueous glucose) were prepared (Table 4). The viscosity of the solution was determined by a tuning fork vibrating viscometer (SV-1A: viscosity measuring range: 0.3 to 10,000 mPa·s, SV-1H: viscosity measurement range: 0.3 to 1,000 mPa·s, A&D Co., Ltd.), measured at room temperature (20°C).

1. Results

Figure 14 shows the relationship between glucose vol% and viscosity. As the volume percent of glucose increased, the viscosity increased exponentially.

Given the glucose volume percent (x) and the viscosity (y), the following relationship (equation 3) was derived:

$$y = 0.898e^{0.0386x}$$

The osmotic pressures in Table 4 were obtained using the Van't Hoff equation (equation 2 above).
The ratio to the osmotic pressure of blood was obtained by converting the osmotic pressure [kPa] of each respective solution into units of mOsm/Kg from the relationship of 1 mOsm/Kg = 2,269.68 Pa, and dividing this value by the osmotic pressure of blood (290 mOsm/kg).

Table 4

| 50% glucose (mL) | 10 | 100 | 1,000 | 2,500 | 5,000 |
|---|---|---|---|---|---|
| Glucose % (v/v) | 0.1 | 1 | 10 | 25 | 50 |
| Viscosity (mPa·s) | 0.88 | 1.01 | 1.28 | 2.23 | 6.40 |
| Temperature | 22 | 22 | 22 | 22 | 22 |
| Osmotic pressure (kPa) | 21 | 209 | 2,093 | 5,232 | 10,465 |
| Ratio to osmotic pressure of blood | 0.0 | 0.3 | 3.2 | 79 | 15.0 |

EXAMPLE 4: Study of the Effective Range of Viscosity and Osmotic Pressure for an Epirubicin-Containing Solution

1. Materials and methods

1) Preparation of solution

Table 5 shows the composition of the solution containing epirubicin.
The solutions are solution C, solution C', solution D, and solution F.
The viscosity values in Table 5 were determined from equation 3. The viscosity of each solution was approximately 1 mPa.s.
The osmotic pressure values in Table 5 were obtained from the Van't Hoff equation (equation 2 above).
The ratio to the osmotic pressure of blood was obtained by converting the osmotic pressure [kPa] of each respective solution into units of mOsm/kg from the relationship of 1 mOsm/kg = 2,269.68 Pa, and dividing this value by the osmotic pressure of blood (290 mOsm/kg).

Table 5

| Drug solution | Solution C' | Solution C | Solution D | Solution F |
|---|---|---|---|---|
| Glucose 50% (μL) | 95.0 | 95.0 | 130.0 | 170.0 |
| Saline (μL) | 0.0 | 0.0 | 0.0 | 0.0 |
| Epirubicin (μL) diluted with PBS | 0 | 100 | 100 | 100 |
| PBS | 505 | 405 | 370 | 330 |
| 250 μg/mL ICG (μL), diluted with PBS | 400 | 400 | 400 | 400 |
| Total (μL) | 1,000 | 1,000 | 1,000 | 1,000 |
| | | | | |
| Glucose vol% (v/v) | 4.8 | 4.8 | 6.5 | 8.5 |
| Viscosity (mPa·s) | 1.08 | 1.08 | 1.15 | 1.25 |
| Temperature | 22 | 22 | 22 | 22 |
| Osmotic pressure (kPa) | 1,600 | 1,600 | 1,943 | 2,335 |
| Ratio to osmotic pressure of blood | 2.4 | 2.4 | 3.0 | 3.5 |

2) Tumor cells

FM3A-Luc murine breast cancer cells expressing the luciferase gene were used (Shao L, Mori S, Yagishita Y, Okuno T, Hatakeyama Y, Sato T, Kodama T. Lymphatic mapping of mice with systemic lymphoproliferative disorder: Usefulness as an inter-lymph node metastasis model of cancer. J Immunol Methods 2013; 389:69-78). Cells were cultured in RPMI-1640 medium (Biological Industries, Haemek, Israel) containing 0.5% G418 (Sigma-Aldrich), 10% fetal bovine serum (FBS; Sigma-Aldrich, St Louis, MO, USA), and 1% L-glutamine-penicillin-streptomycin (Sigma-Aldrich). The culture conditions were 37°C and 5% $CO_2$.

3) Cell inoculation

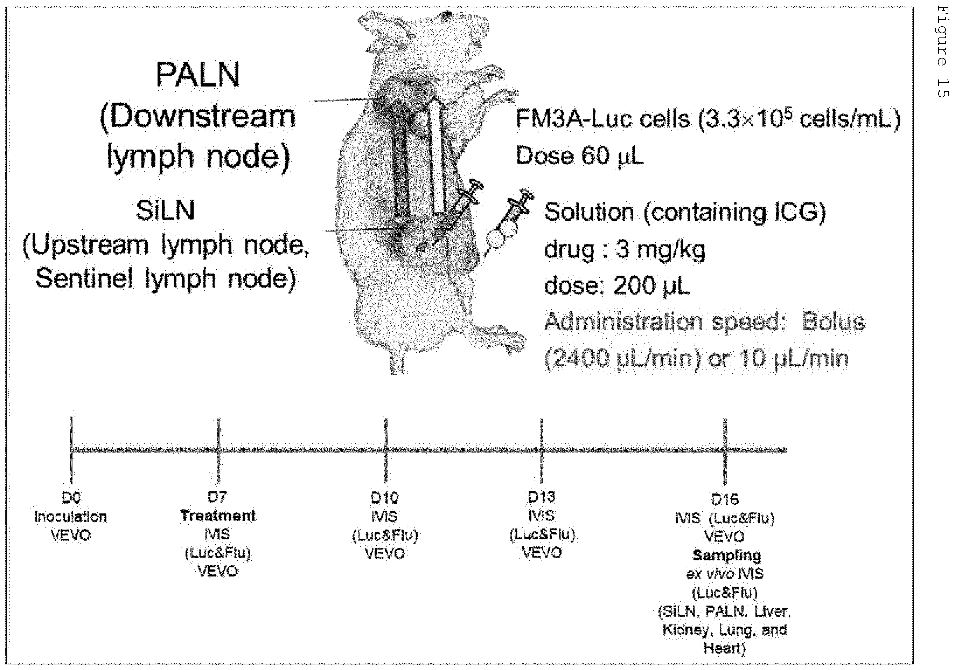

Cell solution ($3.3 \times 10^5$ cells/mL) with a volume of 60 μL was inoculated into the subiliac lymph node (SiLN). This date was defined as Day 0. A schematic diagram of the experimental protocol is shown in Figure 15.

4) Administration of solutions

On the seventh day (D7) after inoculation, 200 μL of epirubicin solution was administered as a bolus injection into the subiliac lymph node and delivered to the proper axillary lymph node (PALN). The drug concentration was 3 mg per kg/mouse, assuming a mouse weighed 34 g.

5) Pathological analysis

On the 16th day (D16) after tumor transplantation, the subiliac lymph node and the proper axillary lymph node were excised, and a pathological evaluation was performed after staining with hematoxylin-eosin.

2. Experimental results

1) Bolus administration

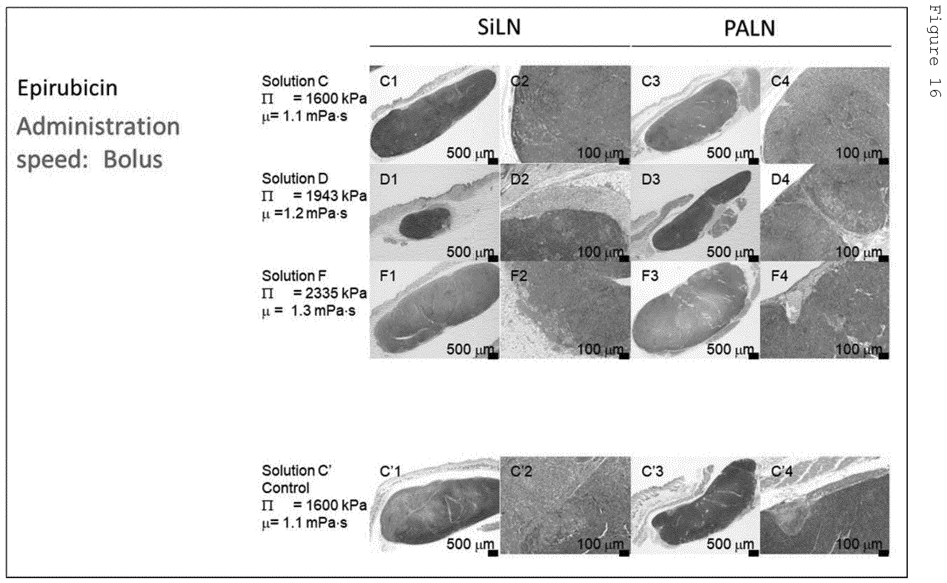

Figure 16 shows a pathological image obtained on Day 16 after tumor cell inoculation.

(C) Solution C

SiLN (C-1, C-2)

No tumor invasion or proliferation was recognized in the lymph node. It may be considered that this is an inhibitory effect on proliferation by the administered drug.

PALN (C-3, C-4)

No tumor cells were recognized in the lymph node. It may be considered that this is an inhibitory effect on metastasis produced by the administered drug.

(D) Solution D

SiLN (D-1, D-2)

There were a few tumor cells that seemed to have infiltrated and proliferated from the marginal sinus of the lymph node, but most of the tumor tissue had become necrotic and replaced by fibrous tissue. It may be considered that this is an inhibitory effect on proliferation by the administered drug.

PALN (D-3, D-4)

No tumor cells were observed in the lymph node. It may be considered that this is an inhibitory effect of the administered drug on metastasis.

(F) Solution F

SiLN (F-1, F-2)

No tumor cells were identified in the lymph node. It may be considered that the administered drug inhibited the growth of tumor cells.

PALN (F-3, F-4)

No tumor cells were detected in the lymph node. It may be considered that the administered drug exerted an inhibitory effect on metastasis.

(C') Solution C', Control

SiLN (C'-1, C'-2)

Tumor cells that seem to have infiltrated and proliferated from the marginal sinus of the lymph node showed a marked tendency to proliferate and form a tumor mass around the lymph node.

PALN (C'-3, C'-4)

Invasion and proliferation of tumor cells were recognized in the marginal sinus of the lymph node, and metastatic lesions were formed.
From the above, it has been shown that the effect of the present invention is exhibited at an osmotic pressure ranging from 1.600 kPa to 2,335 kPa, and an excellent drug effect can be produced using the lymphatic drug delivery method when solutions containing epirubicin are used.

EXAMPLE 5: Study of the Effective Range of Osmotic Pressure for a Nimustine-Containing Solution

1. Materials and methods

1) Preparation of solution

Table 6 shows the compositions of the solutions containing nimustine.
The solutions were solution C and solution D.
The viscosity values in Table 6 were calculated from equation 3.
The viscosity was approximately 1 mPa.s.
The osmotic pressure values in Table 6 were obtained from the Van't Hoff equation (equation 2 above).
The ratio to the osmotic pressure of blood was obtained by converting the osmotic pressure [kPa] of each respective solution into units of mOsm/kg from the relationship of 1 mOsm/kg = 2269.68 Pa, and dividing this value by the osmotic pressure of blood (290 mOsm/kg).

Table 6

| Drug solution | Solution C | Solution D |
|---|---|---|
| Glucose 50% (μL) | 95.0 | 130.0 |
| Saline (μL) | 0.0 | 0.0 |
| Nimustine (μL) diluted with PBS | 100 | 100 |
| PBS | 405 | 370 |
| 250 μg/mL ICG (μL), diluted with PBS | 400 | 400 |
| Total (μL) | 1,000 | 1,000 |
| | | |
| Glucose vol% (v/v) | 4.8 | 6.5 |
| Viscosity (mPa.s) | 1.08 | 1.15 |

(continued)

| Drug solution | Solution C | Solution D |
|---|---|---|
| Osmotic pressure (kPa) | 1,600 | 1,943 |
| Ratio to osmotic pressure of blood | 2.4 | 3.0 |

2) Tumor cells

FM3A-Luc murine breast cancer cells expressing the luciferase gene were used (Shao L, Mori S, Yagishita Y, Okuno T, Hatakeyama Y, Sato T, Kodama T. Lymphatic mapping of mice with systemic lymphoproliferative disorder: Usefulness as an inter-lymph node metastasis model of cancer. J Immunol Methods 2013; 389:69-78). Cells were cultured in RPMI-1640 medium (Biological Industries, Haemek, Israel) containing 10% fetal bovine serum (FBS; Sigma-Aldrich, St Louis, Mo., US), 1% L-glutamine-penicillin-streptomycin (Sigma-Aldrich), and 0.5% G418 (Sigma-Aldrich). The culture conditions were 37°C and 5% $CO_2$.

3) Cell inoculation

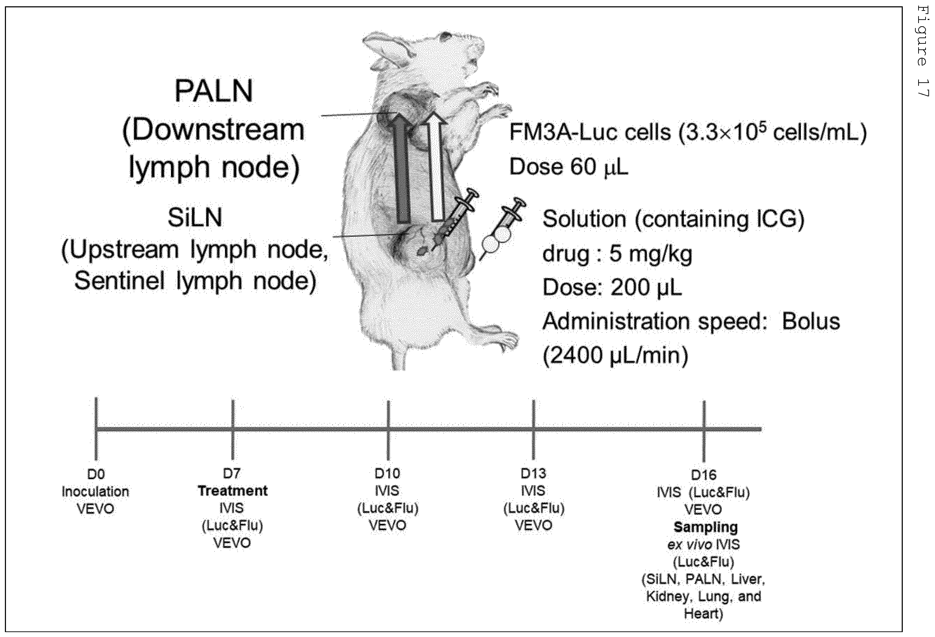

Cell solution ($3.3 \times 10^5$ of cells/mL) with a volume of 60 $\mu$L was inoculated into the subiliac lymph node. This date was defined as Day 0. Figure 17 shows a conceptual diagram of an experiment with nimustine.

4) Solution administration

On the seventh day (D7) after cell inoculation, 200 $\mu$L of nimustine solution was administered as a bolus injection into the subiliac lymph node and delivered to the proper axillary lymph node. Drug concentrations were 5 mg per kg/mouse, assuming a mouse weighed 34 g.

5) Pathological analysis

On the 16th day (D16) after tumor transplantation, the subiliac lymph node and the proper axillary lymph node were excised, and a pathological evaluation was made after staining with hematoxylin-eosin.

2. Experimental results

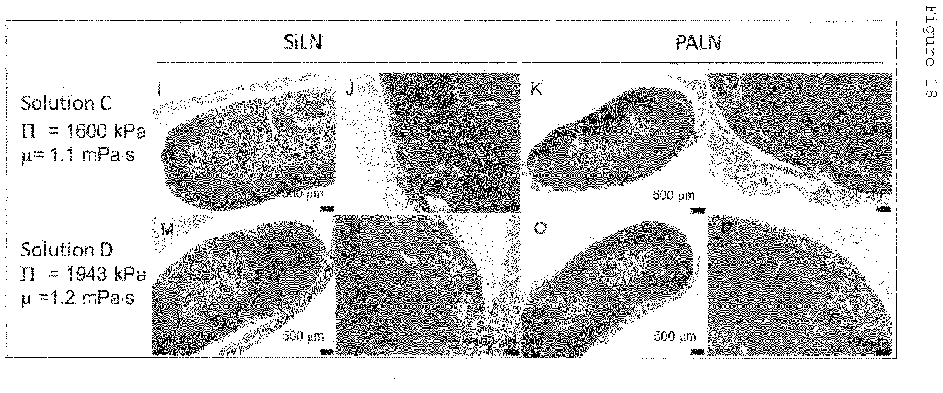

Figure 18 is a pathological image (bolus administration of nimustine-containing solution) taken 16 days after inoculation of tumor cells.

(C) Solution C

SiLN (I, J)

No tumor cells were seen in the lymph node. It may be considered that the administered drug had exerted an inhibitory effect on tumor growth.

PALN (K, L)

No tumor cells were observed in the lymph node. A potential effect of the administered drug to inhibit metastasis may be considered.

(D) Solution D

SiLN (M, N)

No tumor cells were seen in the lymph node. It may be considered that the administered drug had exerted an inhibitory effect on the growth of tumor.

PALN (O, P)

No tumor cells were seen in the lymph node. An effect of the administered drug to inhibit metastasis may be considered.

From the above findings, the effect of the present invention was demonstrated, and excellent drug effects were exerted when the lymphatic drug delivery method was used with solutions containing nimustine at an osmotic pressure ranging from 1,600 kPa to 1,943 kPa.

EXAMPLE 6: Study of the Effective Range of Osmotic Pressure for a Methotrexate-Containing Solution

1. Materials and methods

1) Preparation of solution

Table 7 shows the composition of the solution containing methotrexate.
The solutions were solution B, solution C and solution D.
The viscosity values in Table 7 were determined from equation 3.
The viscosity was approximately 1 mPa.s.
The osmotic pressure values in Table 7 were obtained from the Van't Hoff equation (equation 2).
The ratio to the osmotic pressure of blood was obtained by converting the osmotic pressure [kPa] of each respective solution into units of mOsm/kg from the relationship of 1 mOsm/kg = 2,269.68 Pa and dividing this value by the osmotic pressure of blood (290 mOsm/kg).

Table 7

| Drug solution | Solution B | Solution C | Solution D |
|---|---|---|---|
| Glucose 50% ($\mu$L) | 55.0 | 95.0 | 130.0 |
| Saline ($\mu$L) | 0.0 | 0.0 | 0.0 |
| Methotrexate ($\mu$L) diluted with PBS | 100 | 100 | 100 |
| PBS | 445 | 405 | 370 |
| 250 $\mu$g/mL ICG ($\mu$L), diluted with PBS | 400 | 400 | 400 |
| Total ($\mu$L) | 1,000 | 1,000 | 1,000 |
| | | | |
| Glucose vol% (v/v) | 2.8 | 4.8 | 6.5 |
| Viscosity (mPa·s) | 1.00 | 1.08 | 1.15 |
| Osmotic pressure (kPa) | 1,208 | 1,600 | 1,943 |
| Ratio to osmotic pressure of blood | 1.8 | 2.4 | 3.0 |

2) Tumor cells

FM3A-Luc murine breast cancer cells expressing the luciferase gene were used (Shao L, Mori S, Yagishita Y, Okuno T, Hatakeyama Y, Sato T, Kodama T. Lymphatic mapping of mice with systemic lymphoproliferative disorder: Usefulness as an inter-lymph node metastasis model of cancer. J Immunol Methods 2013; 389:69-78). Cells were cultured in RPMI-1640 medium (Biological Industries, Haemek, Israel) containing 10% fetal bovine serum (FBS; Sigma-Aldrich, St Louis, Mo., US), 1% L-glutamine-penicillin-streptomycin (Sigma-Aldrich) and 0.5% G418 (Sigma-Aldrich). The culture conditions were 37°C and 5% $CO_2$.

3) Cell inoculation

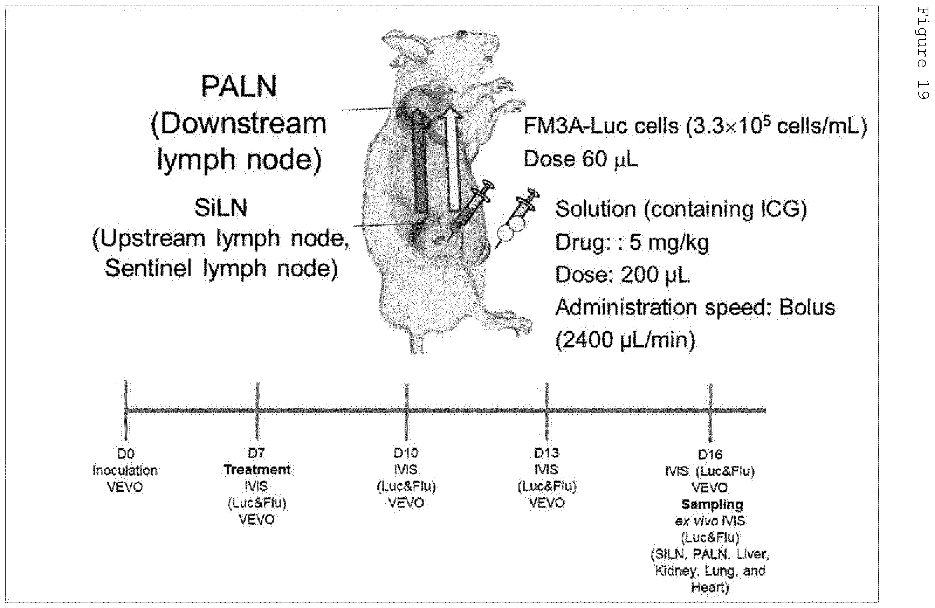

Cell solution ($3.3 \times 10^5$ cells/mL) with a volume of 60 $\mu$L was injected into the subiliac lymph node. This date was taken as Day 0. Figure 19 shows a conceptual diagram of the methotrexate experiment.

4) Solution administration

On the seventh day after cell inoculation (D7), 200 μL of methotrexate solution was administered to the subiliac lymph node as a bolus and delivered to the proper axillary lymph node. Drug concentrations were 5 mg per kg/mouse, assuming a mouse weighed 34 g.

5) Pathological analysis

On the 16th day (D16) after tumor transplantation, the subiliac lymph node and the proper axillary lymph node were excised, and a pathological evaluation was made after staining with hematoxylin-eosin.

2. Experimental results

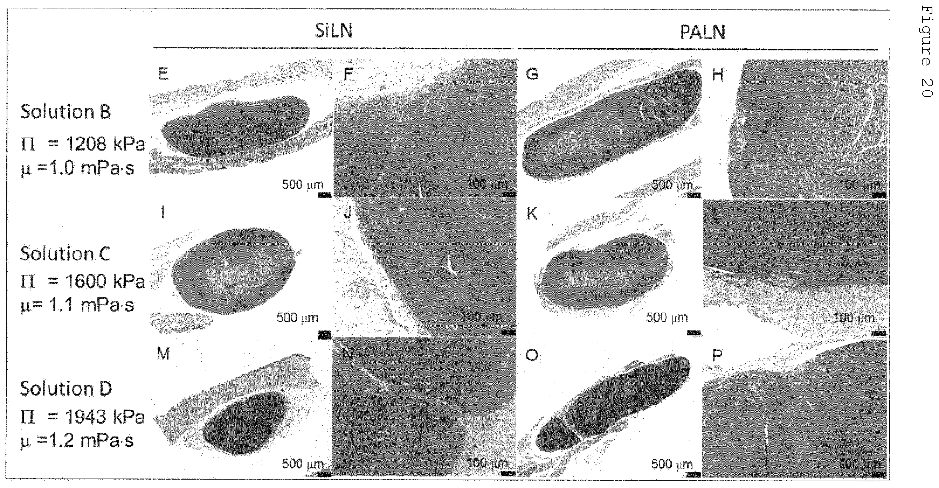

The pathological evaluations of the antitumor effects of the methotrexate-containing solutions are shown in Figure 20.

(B) Solution B

SiLN (E, F)

No obvious growth of tumor cells in the lymph node was evident. It may be considered that the administered drug exerted an inhibitory effect on tumor growth.

PALN (G, H)

No obvious growth of tumor cells in the lymph node was detected. An effect of the administered drug to inhibit metastasis may be considered.

(C) Solution C

SiLN (I, J)

No obvious growth of tumor cells occurred in the lymph node. It may be considered that the administered drug exerted an inhibitory effect on tumor growth.

PALN (K, L)

No obvious growth of tumor cells in the lymph node was detected. An effect of the administered drug to inhibit metastasis may be considered.

(D) Solution D

SiLN (M, N)

No obvious growth of tumor cells in the lymph node was detected. It may be considered that the administered drug exerted an inhibitory effect on tumor growth.

PALN (O, P)

No obvious growth of tumor cells in the lymph node was found. An effect of the administered drug to inhibit metastasis may be considered.
The above findings demonstrate that the effect of the present invention is exhibited at an osmotic pressure of 1,208 kPa to 1,943 kPa, and a good drug effect is obtained using the lymphatic drug delivery method with a solution containing methotrexate.

Industrial applicability

The intralymphatic preparation according to the present invention facilitates the administration of a drug to a lymph node, thereby delivering the drug efficiently to the lymph node downstream from the administered lymph node. For example, when the drug is an anticancer drug, not only will the anticancer effect be exerted on the target lymph node, but the anticancer drug can efficiently flow into other lymph nodes to which micro-cancers may have spread, and their recurrence can thus be prevented.

For example, if cancer is present in a lymph node in an area that cannot be dissected by surgery, it is not possible to cure the cancer by surgery. However, by using an intralymphatic preparation of the present invention, it is possible to deliver an anticancer drug from an upstream lymph node to treat the lymph node in an area that cannot be dissected. In the intralymphatic preparation of the present invention, the amount of drug used is much less than that used during conventional systemic administration, and therefore, the drug produces fewer adverse effects and is much safer for the patient.

**Claims**

1. An intralymphatic liquid preparation for delivering a drug to a target lymph node by a lymphatic drug delivery method, the preparation comprising a drug, wherein the osmotic pressure of the liquid ranges from 700 to 2,700 kPa.

2. The preparation according to claim 1, wherein the osmotic pressure of the liquid is $\geq$ 900 kPa.

3. The preparation according to claim 1, wherein the osmotic pressure of the liquid is $\leq$ 2,400 kPa.

4. The preparation according to claim 1, wherein the osmotic pressure of the liquid ranges from 950 to 2,000 kPa.

5. The preparation according to any one of claims 1 to 4, wherein the viscosity of the liquid ranges from 0.5 to 20 mPa·s.

6. The preparation according to any one of claims 1 to 4, wherein the viscosity of the liquid ranges from 1.0 to 15 mPa·s.

7. The preparation according to any one of claims 1 to 6, wherein the preparation comprises a nonionic surfactant.

8. The preparation according to claim 7, wherein the nonionic surfactant is a polyoxyethylene sorbitan fatty acid ester.

9. The preparation according to claim 8, wherein the polyoxyethylene sorbitan fatty acid ester is polyoxyethylene sorbitan oleate.

10. The preparation according to any one of claims 1 to 9, wherein the drug is a pharmaceutically active substance, a nucleic acid molecule container or a cultured cell.

11. The preparation according to any one of claims 1 to 9, wherein the drug is an anticancer drug.

Figure 1

Figure 2

Figure 3

Figure 4

Figure 5

Figure 6

Figure 7

Figure 8

Figure 9

Figure 10

Figure 11

Figure 12

Figure 13

Weight changes

Figure 14

$y = 0.898e^{0.0386x}$

$R^2 = 0.9954$

Figure 15

PALN (Downstream lymph node)

SiLN (Upstream lymph node, Sentinel lymph node)

FM3A-Luc cells ($3.3 \times 10^5$ cells/mL)

Dose 60 μL

Solution (containing ICG)

drug : 3 mg/kg

dose: 200 μL

Administration speed:  Bolus (2400 μL/min) or 10 μL/min

D0
Inoculation
VEVO

D7
Treatment
IVIS
(Luc&Flu)
VEVO

D10
IVIS
(Luc&Flu)
VEVO

D13
IVIS
(Luc&Flu)
VEVO

D16
IVIS (Luc&Flu)
VEVO
Sampling
ex vivo IVIS
(Luc&Flu)
(SiLN, PALN, Liver, Kidney, Lung, and Heart)

Figure 16

Figure 17

Figure 18

Figure 19

Figure 20

## INTERNATIONAL SEARCH REPORT

| International application No. |
| --- |
| PCT/JP2018/032220 |

A.  CLASSIFICATION OF SUBJECT MATTER
Int.Cl.  A61K47/26(2006.01)i, A61K9/08(2006.01)i, A61K31/282(2006.01)i,
          A61K31/505(2006.01)i, A61K31/519(2006.01)i, A61K31/704(2006.01)i,
          A61K31/7088(2006.01)i, A61K33/24(2006.01)i, A61K35/12(2015.01)i,
          A61K45/00(2006.01)i, A61P35/00(2006.01)i, A61P35/04(2006.01)i
According to International Patent Classification (IPC) or to both national classification and IPC

B.  FIELDS SEARCHED

Minimum documentation searched (classification system followed by classification symbols)
Int.Cl.  A61K47/26, A61K9/08, A61K31/282, A61K31/505, A61K31/519, A61K31/704,
          A61K31/7088, A61K33/24, A61K35/12, A61K45/00, A61P35/00, A61P35/04

Documentation searched other than minimum documentation to the extent that such documents are included in the fields searched
Published examined utility model applications of Japan          1922–1996
Published unexamined utility model applications of Japan        1971–2018
Registered utility model specifications of Japan                1996–2018
Published registered utility model applications of Japan        1994–2018

Electronic data base consulted during the international search (name of data base and, where practicable, search terms used)
JSTPlus/JMEDPlus/JST7580 (JDreamIII)
CAplus/MEDLINE/EMBASE/BIOSIS (STN)

C.  DOCUMENTS CONSIDERED TO BE RELEVANT

| Category* | Citation of document, with indication, where appropriate, of the relevant passages | Relevant to claim No. |
| --- | --- | --- |
| X<br><br>A | 藤井穂乃香 ほか，　リンパネットワークを利用した転移リンパ節治療法の開発，In: 第29回バイオエンジニアリング講演会講演論文集，18 January 2017, in particular, column "2B42", (FUJII, Honoka et al., "Development of a treatment for metastatic lymph nodes via lymphatic network", Proceedings of the 29th lecture conference of Bioengineering) | 1, 3, 5, 6, 10, 11<br>2, 4, 7-9 |
| A | JP 2015-192670 A (TOHOKU UNIVERSITY) 05 November 2015 (Family: none) | 1-11 |
| A | MIURA, Yoshinobu et al., "Early diagnosis of lymph node metastasis: Importance of intranodal pressures", Cancer Science, 2016, vol. 107, no. 3, pp. 224-232, ISSN: 1349-7006 | 1-11 |

☐  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 21 November 2018 (21.11.2018) | 04 December 2018 (04.12.2018) |

| Name and mailing address of the ISA/<br>    Japan Patent Office<br>    3-4-3, Kasumigaseki, Chiyoda-ku,<br>    Tokyo 100-8915, Japan | Authorized officer<br><br><br>Telephone No. |
| --- | --- |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHAO L ; MORI S ; YAGISHITA Y ; OKUNO T ; HATAKEYAMA Y ; SATO T ; KODAMA T.** Lymphatic mapping of mice with systemic lymphoproliferative disorder: usefulness as an inter-lymph node metastasis model of cancer. *J Immunol Methods,* 2013, vol. 389, 69-78
- **MIKADA M ; SUKHBAATAR A ; MIURA Y ; HORIE S ; SAKAMOTO M ; MORI S ; KODAMA T.** Evaluation of the enhanced permeability and retention effect in the early stages of lymph node metastasis. *Cancer Sci,* 2017, vol. 108, 846-852
- **KODAMA T ; HATAKEYAMA Y ; KATO S ; MORI S.** Visualization of fluid drainage pathways in lymphatic vessels and lymph nodes using a mouse model to test a lymphatic drug delivery system. *Biomed Opt Express,* 2015, vol. 6, 124-34

- **KODAMA T ; MATSUKI D ; TADA A ; TAKEDA K ; MORI S.** New concept for the prevention and treatment of metastatic lymph nodes using chemotherapy administered via the lymphatic network. *Sci Rep,* 2016, vol. 6, 32506
- National Astronomical Observatory. Science Data. Maruzen Co., Ltd, 1997
- **SHAO L ; MORI S ; YAGISHITA Y ; OKUNO T ; HATAKEYAMA Y ; SATO T ; KODAMA T.** Lymphatic mapping of mice with systemic lymphoproliferative disorder: Usefulness as an inter-lymph node metastasis model of cancer. *J Immunol Methods,* 2013, vol. 389, 69-78
- *J Immunol Methods,* 2013, vol. 389, 69-78
- **SHAO L ; MORI S ; YAGISHITA Y ; OKUNO T ; HATAKEYAMA Y ; SATO T ; KODAMA T.** Lymphatic mapping of mice with systemic lymphoproliferative disorder: Usefulness as an inter-lymph node metastasis model of cancer. *J Immunol Methods,* 2013, vol. 389, 69-78